# EUROPÄISCHE PATENTANMELDUNG

(11) **EP 2 397 102 A1**
(43) Veröffentlichungstag der Anmeldung: **21.12.2011**
(21) Anmeldenummer: 11006938.2
(22) Anmeldetag: 22.08.2007
(51) Int. Cl.: A61C 1/08, A61C 8/00

(54) **Bohrerführung**

(62) Teilanmeldung aus: 07016398.5
(71) Anmelder: Straumann Holding AG, 4002 Basel (CH)
(72) Erfinder: Suter, Edmund, 4002 Basel (CH); Streff, Patrick, 4002 Basel (CH)
(74) Vertreter: Hoffmann, Cornelia

(57) **Zusammenfassung**

Die vorliegende Erfindung betrifft eine Bohrerführung zur Verwendung im Dentalbereich. Die Bohrerführung umfasst einen ein- oder mehrteiligen Griffteil und wenigstens zwei Führungshülsen mit jeweils einem oberen Ende und einem unteren Ende, wobei der Griffteil eine Oberseite und eine Unterseite aufweist. Die Unterseite des Griffteils ist zumindest teilweise dazu bestimmt, auf einer Bohrschablone aufzuliegen. Die wenigstens zwei Führungshülsen stehen an der Unterseite des Griffteils vor. Die Innendurchmesser der wenigstens zwei Führungshülsen entsprechen jeweils zwei Standarddurchmessem von Bohrern für Dentalimplantate mit Durchmessern zwischen 2 und 5 mm in beliebigen Abständen, und ein Chirurg kann durch einfaches Drehen der Bohrerführung (1) eine Führungshülse (10) mit grösserem Innendurchmesser in die Bohrschablone einführen.

## Beschreibung

Die vorliegende Erfindung betrifft eine Bohrerführung zur Verwendung im Dentalbereich.

Zum Erzielen einer prothetisch optimierten lmplantatachse wird häufig eine Bohrschablone, z.B. eine OP-Schabtone/CT-Schiene eingesetzt. Bekannt sind Bohrschablonen zum Führen von Dentalimplantatbohrem zur Aufbereitung der Kieferknochen, welche zur Aufnahme eines oder mehreren Dentalimplantaten dienen sollen. Derartige Bohrschablonen werden manuell modelliert oder computergesteuert hergestellt (CAM). Sie weisen Bohrlöcher auf, die zur Führung eines Dentalimplantatbohrers oder des Implantates während eines operativen Eingriffs dienen. Oft werden in die Bohrschablonen standardisierte Metallbohrhülsen (nachfolgend Standardbohrhülsen) eingearbeitet, z.B. einpolymerisiert oder eingepresst, um die Präzision der Bohrungen zu erhöhen. Während eines operativen Eingriffs, wird der Dentalimplantatbohrer durch eine derartige Hülse geführt. Die Bohrschablone dient somit dazu, die Bohrung präzise gemäss den aus der Planung ermittelten optimalen Implantatachsen umzusetzen.

Bei den meisten Implantationsverfahren werden die Bohrungen in mehreren Schritten erarbeitet. Beispielsweise erfolgt eine erste Bohrung mit einem Pilotbohrer geringen Durchmessers, gefolgt von einer Bohrung mit einem Spiralbohrer mit dem definitiven Durchmessers des zu setzenden Implantats. Es können je nach Verfahren auch weitere Bohrer mit Zwischendurchmessem oder anderen Schneidgeometrien erforderlich sein.

Um dieses Verfahren anzuwenden, werden entsprechende Reduktionshülsen, die den Durchmesser der Bohrung definieren, in die Löcher oder Standardbohrhülsen der Bohrschablonen eingelegt.

Da die Reduktionshülsen auch während der Behandlung ausgetauscht werden und diese typischerweise Durchmesser von weniger als 6mm haben, ergeben sich Schwierigkeiten bei der Handhabung oder gar die Gefahr der Aspiration durch den Patienten.

Aus WO 06/130067 sind Reduktionshülsen mit griffartigen Verlängerungen bekannt. Die schrittweise Durchmesserreduktion wird durch Ineinanderstapeln mehrerer Reduktionshülsen erreicht.

In WO 06/014130 wird eine Reduktionshülse beschrieben, die über ein Kugelgelenk mit einem Griffteil verbunden ist, um verschiedenen Platzsituationen im Patientenmund gerecht werden zu können.

WO 97/49351 offenbart eine implantatgestützte Vorrichtung zur Bohrerführung mit einem Hilfseinsatz, aus dem zwei Reduktionshülsen gleichen Durchmessers ragen, die über eine flache Verbindung in paralleler Position gehalten werden.

Der vorliegenden Erfindung liegt die Aufgabe zugrunde, ein einfach zu handhabendes Hilfsmittel zur Durchmesserreduktion der Löcher in Bohrschablonen bereitzustellen. Die Aufgabe wird durch eine Bohrerführung mit den Merkmalen von Anspruch 1 gelöst. Weitere bevorzugte Ausführungsformen sind Gegenstand der Ansprüche 2 bis 16.

Durch die erfindungsgemässe Bohrerführung ist es möglich, mit einer Vielzahl von verschiedenen Bohrern in einer Bohrschablone zu arbeiten, ohne dass eine komplizierte Handhabung von kleinen Reduktionshülsen nötig ist. Der Chirurg kann durch einfaches Drehen der erfindungsgemässen Bohrerführung eine Führungshülse mit grösserem Innendurchmesser in die Bohrschablone einführen. Mit einem abgestimmten Satz von zwei erfindungsgemässen Bohrerführungen werden vier verschiedene Bohrerdurchmesser abgedeckt, was für die meisten Verfahren zur Herstellung einer Implantatbohrung ausreichend ist.

Die erfindungsgemässe Bohrerführung umfasst einen ein- oder mehrteiligen Griffteil und wenigstens zwei Führungshülsen mit jeweils einem oberen Ende und einem unteren Ende. Der Griffteil weist eine Oberseite und eine Unterseite auf. Die wenigstens zwei Führungshülsen an der Unterseite des Griffteils stehen vor, d.h. sie sind auf der gleichen Seite des Griffteils angeordnet. Dadurch ist die Bohrerführung weniger sperrig und kann auch bei einer schwierigen Platzsituation im Patientenmund einfach zum Einsatz kommen.

Der Griffteil kann starr oder formbar sein. Ebenso kann er flach, halbrund oder rund sein. Um eine bessere Sterilisierbarkeit zu gewährleisten, ist die Oberfläche bevorzugt glatt. Die Länge der erfindungsgemässen Bohrerführung ist derart gewählt, dass die potentiellen Bohrstellen im Kieferbereich erreicht werden und dabei der Griffteil in der Regel ausserhalb der Mundhöhle gefasst werden kann. Das bevorzugte Griffteil der erfindungsgemässen Bohrerführung hat eine Länge zwischen 5 und 12 cm.

Die Unterseite des Griffteils ist zumindest teilweise dazu bestimmt, auf einer Bohrschablone aufzuliegen. Dadurch erhält der Zahnarzt während dem Bohren noch eine zusätzliche Stabilität, dass der Bohrer nicht rutschen kann. Bevorzugt liegt dabei dieser Auflageteil unmittelbar zur Führungshülse benachbarte auf der Bohrschablone auf, Dies kann beispielsweise die radial nach aussen gehende Fortsetzung der Führungshülse am Griffteil sein oder aber auch ein Endstück des Griffteils.

Die Oberseite des Griffteils neben der Führungshülse dient als Bohrstopp für den Bohrer, d.h. sie stellt sicher, dass der Chirurg nicht zu tief bohrt. Dadurch werden Verletzungen von Nerven beim Bohren vermieden.

Die Führungshülsen, die an der Unterseite des Griffteils vorstehen, weisen ein oberes und ein unteres Ende auf. Das obere Ende ist zu dem Griffteil gerichtet, während das untere Ende auf der von der Unterseite des Griffteils abgewandten Seite angeordnet ist. Es ist möglich die Führungshülsen separat zu fertigen und diese in den Griffteil einzufügen, wobei das obere Ende der Führungshülse mit dem Griffteil verbunden wird. Bevorzugt wird jedoch die Bohrerführung einstückig gefertigt, um eine bessere Sterilisierbarkeit sicherzustellen. Die Führungshülsen sind zylindrisch und weisen einen solchen Aussendurchmesser auf, dass sie passgenau in die Bohrlöcher der Bohrschablone respektive die entsprechenden Standardbohrhülsen passen. Der Innendurchmesser ist auf die Durchmesser der Bohrer abgestimmt. In einer bevorzugten Ausführungsform weist die erfindungsgemässe Bohrerführung wenigstens zwei Führungshülsen auf, deren Innendurchmesser jeweils zwei Standarddurchmesser von Bohrern für Dentalimplantate mit Durchmessern zwischen 2 und 5 mm in beliebigen Abständen entspricht. Ein Satz von zwei Bohrerführungen mit je zwei Führungshülsen abgestimmt auf die Bohrerdurchmesser von 2.2, 2.8, 3.5, und 4.2 mm steilen eine bevorzugte Ausführungsform dar.

Die Höhe der Führungshülsen bestimmt zusammen mit der Bohrergeometrie die Präzision der Bohrung. Lange Führungshülsen erhöhen zwar die Präzision der Bohrerführung, gleichzeitig aber auch die Gesamthöhe der aufeinanderliegenden Teile der Bohrschablone, Bohrerführung und Bohrergerät. Befriedigende Ergebnisse werden mit Führungshülsen ab 5 mm Höhe im zylindrischen Bereich erreicht. Idealerweise beträgt die Höhe aber nicht mehr als 10 mm.

Die Führungshülsen können entweder jeweils endständig an jedem Ende des Griffteils angeordnet werden oder aber es ist möglich mehrere Führungshülsen an einem Ende des Griffteils anzuordnen. Letztere Ausführungsform weist vorzugsweise Sollbruchstellen zwischen den Führungshülse auf und kann vorteilhaft als Einwegbohrerführung verwendet werden.

Instrumente in der dentalen lmplantologie müssen im besonderen Masse die Dimensionen und die Physiologie im Patientenmund berücksichtigen. Werden zwei Führungshülsen über ein Griffteil verbunden, sollten sie derart angeordnet sein, dass das zweite Ende der Bohrerführung, das sich nicht in der Bohrschablone befindet, nicht den Einsatz der in der Bohrschablone einzusetzenden ersten Führungshülse behindert. Nebst der Tatsache, dass die Führungshülsen auf der gleichen Seite des Griffteils angeordnet sind, ist es von Vorteil, wenn der Griffteil abgewinkelt ist. In einer bevorzugten Ausführungsform sind beispielsweise beide Enden des Griffteils s-förmig, sodass das Griffteil eine brückenartige Form aufweist. Dadurch hat die Zunge des Patienten darunter ausreichend Platz und wird durch das darüber liegenden Griffteil von der Bohrstelle ferngehalten. Zudem ist die Bohrerführung vorzugsweise derart geformt sein, dass sie sich auch im teilbezahnten Kiefer problemlos an jeder Stelle einsetzen lässt. Daher sollte die s-förmige Abwinkelung im Griffteil möglichst nahe bei der Führungshülse liegen. Gleichzeitig muss über der Führungshülse ausreichend Platz für das Kopfstück der gebräuchlichen Dentalbohrvorrichtungen bleiben. Die Führungshülsen der vorliegenden Erfindung stehen vorzugsweise parallel zueinander am Griffteil, weil dadurch die Gesamthöhe der Bohrerführung kleiner bleibt. Es ist jedoch auch eine Abwinkelung des Griffteils denkbar, wobei diese Abwinkelung vorzugsweise im mittleren Teil des Griffteils angeordnet ist. Durch eine solche Abwinkelung stehen die Führungshülsen ebenfalls abgewinkelt zueinander.

Bei der Verwendung von mehreren efindungsgemässen Bohrerführungen kann eine Kodierung verwendet werden. Die häufig auf den Bohrern verwendeten Farbcodes können für die Bohrerführungen übernommen und an geeigneter Stelle, etwa am Griffteil nahe der entsprechenden Führungshülse, aufgebracht werden. Alternativ kann die Führungshülse selbst in der entsprechenden Farbe eingefärbt werden.

Oparationsinstrumente in der Chirurgie müssen zuverlässig gereinigt und sterilisiert werden können. Es werden deshalb einteilige Bohrerführungen mit glatten, zugänglichen Oberflächen bevorzugt. Besonders bevorzugt werden einteilige einstückige Bohrerführungen, da sie auch keine Verbindungsstellen aufweisen. Mehrstückige Bohrerführungen, deren Verbindungsstellen keine Ritzen aufweisen und einfach zu reinigen sind, sind jedoch ebenfalls denkbar. Ebenfalls vorteilhaft ist es während einer Behandlung Operationsinstrumente verwenden zu können, die weder verstellt, umgebaut noch ausgetauscht werden müssen. Die erfindungsgemässe Bohrerführung werden diesen Anforderungen gerecht. Sie weisen keine Hinterschnitte auf und lassen sich leicht sterillsieren.

Die bevorzugten Materialen, aus denen die Bohrerführung der vorliegenden Erfindung gefertigt sind, umfassen rostfreien Stahl, Titan oder andere in der Chirurgie gängige Metalllegierungen. Um die Langlebigkeit der Bohrerführung zu erhöhen, können Teile der Bohrerführung, wie etwa die Führungshülsen, zusätzlich mit oberflächenhärtenden Verfahren behandelt werden. Ein zweckmässiges Verfahren zur Härtung von rostfreiem Stahl ist Kolsterisieren.

Die erfindungsgemässe Bohrerführung kann in Sets zusammen mit Bohrschablonen zur Verfügung gestellt werden. Ebenso ist es möglich, in einem Set mehrer Bohrerführungen mit unterschiedlichen Innendurchmesser zur Verfügung zu stellen.

Die erfndungsgemässe Bohrerführung wird nachfolgend im Detail anhand von bevorzugten Ausführungsformen in den Figuren 1 bis 5 weiter erläutert.
Fig. 1 zeigt eine erfindungsgemässe Bohrerführung mit zwei Führungshülsen.
Fig. 2 zeigt eine Bohrerführung mit einem formbaren Griffteil.
Fig. 3 zeigt eine Bohrerführung mit mehr als zwei Führungshülsen mit verstellbarem Griffteil.
Fig. 4 zeigt eine Bohrschablone bei eingesetzter Bohrerführung mit Dentalbohrer.
Fig. 5 zeigt eine Bohrerführung mit mehreren Führungshülsen an einem Ende des Griffteils.

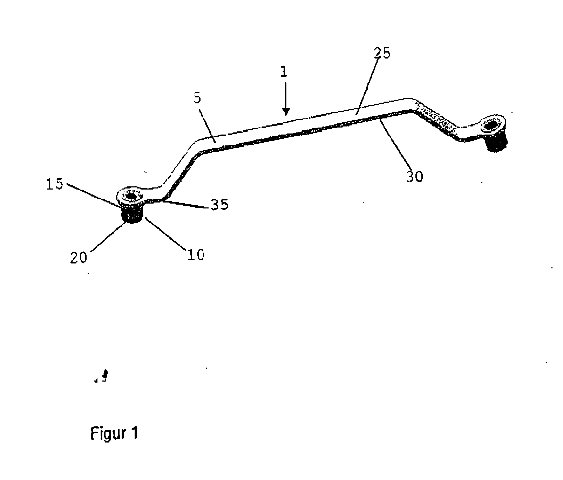

In Figur 1 wird eine Bohrerführung 1 zur Verwendung im Dentalbereich beschrieben. Die Bohrerführung 1 weist einen einteiligen Griffteil 5 und wenigstens zwei Führungshülsen 10 mit jeweils einem oberen zum Griffteil gerichteten Ende 15 und einem unten vom Griffteil abgewandten Ende 20 auf. Der Griffteil weist eine Oberseite 25 und eine Unterseite 30 auf, wobei ein Teil 35 der Unterseite 30 dazu bestimmt ist, auf der Bohrschablone aufzuliegen. Dieser Teil 35, der in direkter Nachbarschaft zu der Führungshülse 10 steht, gewährleistet den Sitz der Bohrerführung auf der Bohrschablone. Die wenigstens zwei Führungshülsen 10 stehen an der Unterseite 30 des Griffteils vor und sind jeweils am Ende des Griffteils angeordnet. Dadurch, dass die Führungshülse gleichgerichtet sind, d.h. an der gleichen Seite des Griffteils angeordnet sind, ist es möglich, für die Zunge im Mundraum möglichst viel Raum zu lassen und dem Patienten einen angenehmen Öffnungswinkel des Mundes während des Bohrvorgangs zu erlauben. Damit die Bohrerführung eingesetzt in die Bohrschablone insgesamt nicht zu weit in den Mundraum wegsteht und dabei eine nachteilige Gesamthöhe erreicht, weist das Griffteil vorzugsweise eine doppelte Abwinkelung oder einen s-förmigen Abschnitt auf, so dass die Bohrerführung eine brückenartige Form hat. Die s-förmige Abwinkelung ist dabei möglichst nahe bei der Führungshülse.

Die in Figur 1 gezeigte Bohrerführung ist einstückig gefertigt und weist keinerlei Hinterschneidungen auf. Durch die Einstückigkeit gibt es keine Verbindungen zwischen einzelnen Komponenten, die es möglicherweise schwerer machen, die Bohrerführung zu sterilisieren. Es ist jedoch auch denkbar, dass die Bohrerführung gemäss Figur 1 mehrstückig gefertigt werden, indem die Führungshülsen separat hergestellt und mit dem Griffteil verbunden werden.

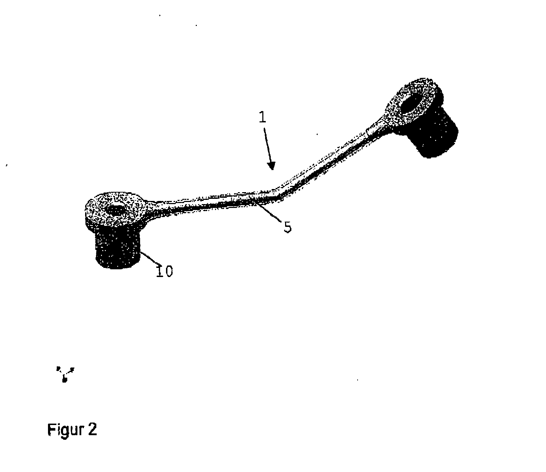

In Figur 2 ist eine zu Figur 1 analoge Bohrerführung dargestellt. Auch sie weist einen Griffteil 5 mit wenigstens zwei Führungshülsen 10 auf. Im Unterschied zu der Bohrerführung in Figur 1 ist der Griffteil 5 der Bohrerführung in Figur 2 abgewinkelt. In einer besonders bevorzugten Ausführungsform ist der Griff von Hand biegbar, was dem Operateur ermöglicht, auch bei sehr schwierigen räumlichen Verhältnissen mit der erfindungsgemässen Bohrerführung zu arbeiten. Dabel kann das Griffteil sowohl ein plastisches als auch ein elastisches Element enthalten. Besonders bevorzugt ist der Griffteil aus einem elastischen Metallkern, der mit einem Plastikmaterial überzogen ist, welches die Griffeigenschaft verbessert. Durch die Abwinkelung des Griffteils, stehen die Achsen der Führungshülsen leicht angewinkelt zu einander. Der Winkel liegt vorzugsweise in einem Bereich von 5 bis 20°.

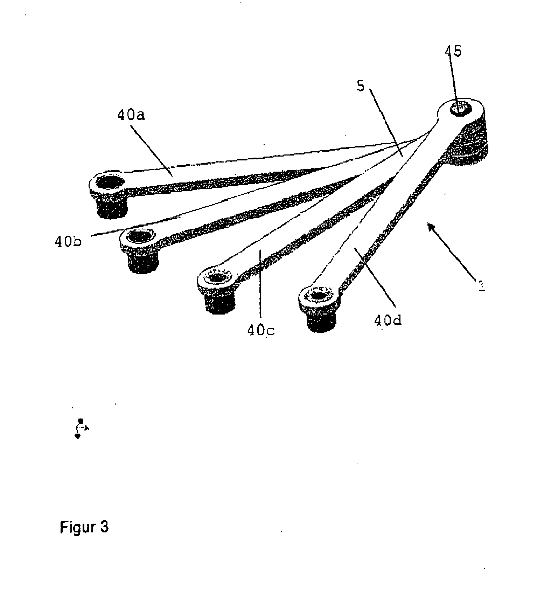

In Figur 3 wird eine weitere bevorzugte Ausführungsform der erfindungsgemässen Bohrerführung dargestellt. Dieser umfasst einen mehrteiligen Griffteil 5, mit verschiedenen Griffabschnitten 40a, 40b, 40c und 40d. Die einzelnen Griffabschnitte sind über eine gemeinsame Achse 45 verbunden, sodass der Griffteil über diese gemeinsame Achse schwenkbar ist. Die einzelnen Griffabschnitte weisen jeweils endständig eine Führungshülse auf. Damit hat der Operateur in einem einzigen Instrument sämtliche Reduktionshülsen, die er benötigt, zur Verfügung. In einer besonders bevorzugten Ausführungsform haben die Griffteilabschnitte jeweils unterschiedliche Längen, sodass die Führungshülsen aneinander vorbei gedreht werden können.

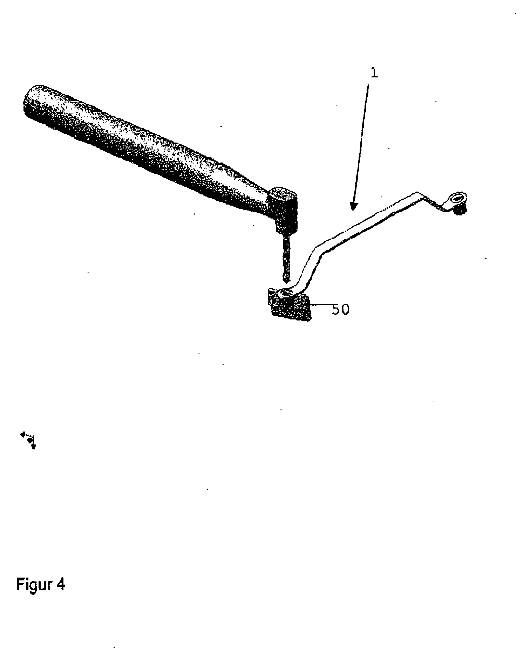

Figur 4 zeigt einen Schnitt durch eine Bohrschablone 50, die die Bohrerführung gemäss Figur 1 enthält.

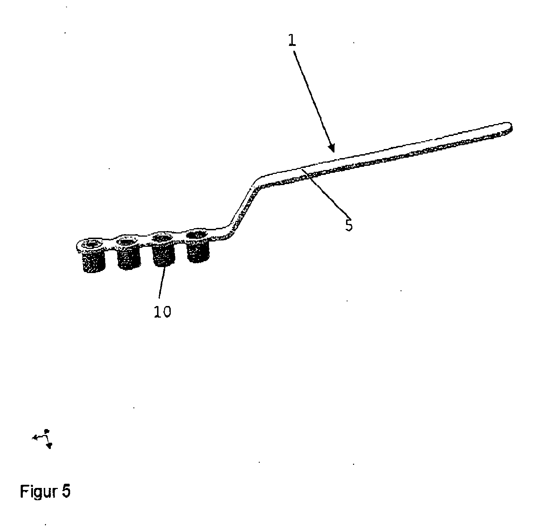

Figur 5 zeigt eine weitere Ausführungsform der vorliegenden Erfindung. Die Bohrerführung 1 weist einen einteiligen Griffteil 5 auf, an dessen einem Ende mehrere Führungshülsen 10 angeordnet sind. Diese Ausführungsform erlaubt ein rationelleres Vorgehen, da die Bohrerführung nicht aus dem Mund entfernt werden muss, wenn eine andere Führungshülse in die Bohrschablone eingeführt wird.

Die übrigen vorteilhaften Eigenschaften der oben für Fig. 1 beschriebenen Bohrerführungen können auch in die Ausführungsformen nach Fig. 2, 3 und 5 übernommen werden.

## Patentansprüche

1. Bohrerführung (1) zur Verwendung im Dentalbereich umfassend einen ein- oder mehrteiligen Griffteil (5) und wenigstens zwei Führungshülsen (10) mit jeweils einem oberen Ende (15) und einem unteren Ende (20), wobei der Griffteil (5) eine Oberseite (25) und eine Unterseite (30) aufweist, und die Unterseite (30) des Griffteils dazu bestimmt ist, zumindest teilweise auf einer Bohrschablone aufzuliegen, wobei die wenigstens zwei Führungshülsen (10) an der Unterseite (30) des Griffteils (5) vorstehen, **dadurch gekennzeichnet, dass** die Innendurchmesser der wenigstens zwei Führungshülsen jeweils zwei Standarddurchmessem von Bohrern für Dentalimplantate mit Durchmessern zwischen 2 und 5 mm in beliebigen Abständen entsprechen und ein Chirurg durch einfaches Drehen der Bohrerführung (1) eine Führungshülse (10) mit grösserem Innendurchmesser in die Bohrschablone einführen kann.

2. Bohrerführung (1) nach Anspruch 1, **dadurch gekennzeichnet, dass** die Bohrerführung (1) einstückig ist.

3. Bohrerführung (1) nach Anspruch 1, **dadurch gekennzeichnet, dass** die Bohrerführung (1) mehrstückig ist.

4. Bohrerführung (1) nach einem der vorangehenden Ansprüche, **dadurch gekennzeichnet, dass** an jedem Ende des Griffteils (5) je eine Führungshülse (10) angeordnet ist.

5. Bohrerführung (1) nach einem der Ansprüche 1 bis 3, **dadurch gekennzeichnet, dass** an einem Ende des Griffteils (5) mehrere Führungshülsen (10) angeordnet sind.

6. Bohrerführung (1) nach einem der vorangehenden Ansprüche, **dadurch gekennzeichnet, dass** der Griffteil (5) abgewinkelt ist.

7. Bohrerführung nach einem der Ansprüche 1 bis 4, **dadurch gekennzeichnet, dass** der Griffteil in der Nähe der Führungshülse einen s-förmigen Abschnitt aufweist.

8. Bohrerführung (1) nach einem der Ansprüche 1 bis 4, **dadurch gekennzeichnet, dass** der Griffteil (5) von Hand biegbar ist.

9. Bohrerführung (1) nach einem der vorangehenden Ansprüche, **dadurch gekennzeichnet, dass** die Führungshülsen (10) parallel zueinander stehen.

10. Bohrerführung (1) nach einem der vorangehenden Ansprüche, **dadurch gekennzeichnet, dass** die Führungshülsen (10) angewinkelt zueinander stehen.

11. Bohrerführung (1) mit einem mehrteiligen Griffteil (5) gemäss Anspruch 1, **dadurch gekennzeichnet, dass** der mehrteilige Griffteil (5) über eine gemeinsame Achse schwenkbar ist.

12. Bohrerführung (1) gemäss einem der vorangehenden Ansprüche aus rostfreiem Stahl.

13. Bohrerführung (1) nach einem der vorangehenden Ansprüche, **dadurch gekennzeichnet, dass** eine Führungshülse (10) einen Innendurchmesser von 3.5 mm und eine Führungshülse (10) einen Innendurchmesser von 4.2 mm aufweist.

14. Kit umfassend eine Bohrschablone (50) mit Bohrlöchern und eine Bohrerführung (1) nach einem der Ansprüche 1 bis 13, **dadurch gekennzeichnet, dass** der Aussendurchmesser der Führungshülse (10) passend zum Durchmesser der Bohrlöcher der Bohrschablone ist.

15. Kit umfassend eine Bohrschablone mit Bohrlöchern enthaltend Standardbohrhülsen und eine Bohrerführung nach einem der Ansprüche 1 bis 13, **dadurch gekennzeichnet, dass** der Aussendurchmesser der Führungshülse passend zum Durchmesser der in den Bohrlöchern der Bohrschablone enthaltenen Standardbohrhülsen ist.

16. Kit umfassend zwei Bohrerführungen nach einem der Ansprüche 1 bis 13, **dadurch gekennzeichnet, dass** sämtliche Führungshülsen unterschiedliche Innendurchmesser aufweisen.
